# EUROPEAN PATENT APPLICATION

(11) **EP 1 182 211 A1**
(43) Date of publication of application: **27.02.2002**
(21) Application number: 00904796.0
(22) Date of filing: 15.02.2000
(51) Int. Cl.: C07K 14/52, C12N 15/19, A61K 38/19

(54) **CHEMOKINE-LIKE FACTORS HAVING CELL CHEMOKINESIS FUNCTION AND PROLIFERATION-STIMULATING ACTIVITY**

(30) Priority: 14.05.1999 CN 99107284
(71) Applicant: Beijing Medical University, Haidian District, Beijing 100083 (CN); Beijing Medical University United Biological Engineering Co., Haidian District, Beijing 100083 (CN)
(72) Inventor: MA, Dalong, Haidian District, Beijing 100083 (CN); HAN, Wenling, Haidian District, Beijing 100083 (CN); ZHANG, Yingmei, Haidian District, Beijing 100083 (CN); SONG, Quansheng, Haidian District, Beijing 100083 (CN); DI, Chunhui, Haidian District, Beijing 100083 (CN); HUANG, Jiaqiang, Haidian District, Beijing 100083 (CN); TANG, Jian, Haidian District, Beijing 100083 (CN); CHEN, Guanghui, Haidian District, Beijing 100083 (CN)
(74) Representative: Reinhard - Skuhra - Weise & Partner
(86) International application number: PCT/CN00/00026
(87) International publication number: WO 00/69910

(57) **Abstract**

The present invention provides a novel chemokine-like factor 1 (CKLF1) polypeptide and variants thereof, which have been identified as having chemotactic and hematopoietic stimulating activities. Also provided are polynucleotides encoding the CKLF1 polypeptide and its variants, and a method to produce such CKLF polypeptides. The present invention further discloses antibodies and antagonists of such CKLF polypeptides. Also disclosed is a medical composition comprising therapeutically effective CKLF polypeptides and pharmaceutically acceptable excipient(s) and carrier(s). Also disclosed are uses of such CKLF polynucleotides and polypeptides in the diagnosis and treatment of immunedeficiency, hematopoietic disorders and primary tumors.

SEQUENCE LISTING

(1)GENERAL INFORMATION:

(i)APPLICANT: BEIJING MEDICAL UNIVERSITY,
BEIJING MEDICAL UNIVERSITY UNITED BIOLOGICAL
ENGINEERING COMPANY

(ii)TITLE OF INVENTION:
CHEMOKINE LIKE FACTOR(CKLF) WITH CHEMOTACTIC
AND HEMATOPOIETIC STIMULATING ACTIVITIES

(iii)NUMBER OF SEQUENCES:8

(iv)ADDRESS:
(A) NAME: DALONG MA
(B) STREET: NO.38 XUANYUAN ROAD, HAIDIAN DISTRICY
(C) CITY: BEIJING
(D) COUNTRY: P.R.CHINA
(E) POSTAL CODE(ZIP): 100083

(v)COMPUTER READABLE FORM:
(A) MEDIUM TYPE: FLOPPY DISK
(B) COMPUTER:PENTIUM 166MMX
(C) OPERATING SYSTEM:WINDOWS 95
(D) SOFTWARE:WORD 97

(1) INFORMATION FOR SEQ ID NO: 1:
(i) SEQUENCE:
(A) LENGTH: 534 BASE PAIRS
(B) TYPE: NUCLEIC ACID
(C) STRANDEDNESS: SINGLE
(D) TOPOLOGY: LINEAR
(ii) MOLECULE TYPE: cDNA
(iii) SEQUENCE DESCRIPTION: SEQ ID NO: 1

3 9 15 21 27 33 39 45
| | | | | | | |
1 GTT CCC AAT CTG AAG TGA AGC CGA GCT GGG CGA GAA GTA GGG GAG
46 GGC GGT GCT CCG CCG CGG TGG CGG TTG CTA TCG CTT CGC AGA ACC
91 TAC TCA GGC AGC CAG CTG AGA AGA GTT GAG GGA AAG TGC TGC TGC
136 TGG GTC TGC AGA CGC GAT GGA TAA CGT GCA GCC GAA AAT AAA ACA
181 TCG CCC CTT CTG CTT CAG TGT GAA AGG CCA CGT GAA GAT GCT GCG
226 GCT GGA TAT TAT CAA CTC ACT GGT AAC AAC AGT ATT CAT GCT CAT
271 CGT ATC TGT GTT GGC ACT GAT ACC AGA AAC CAC AAC ATT GAC AGT
316 TGG TGG AGG GGT GTT TGC ACT TGT GAC AGC AGT ATG CTG TCT TGC
361 CGA CGG GGC CCT TAT TTA CCG GAA GCT TCT GTT CAA TCC CAG CGG
406 TCC TTA CCA GAA AAA GCC TGT GCA TGA AAA AAA AGA AGT TTT GTA
451 ATT TTA TAT TAC TTT TTA GTT TGA TAC TAA GTA TTA AAC ATA TTT
496 CTG TAT TCT TCC AAA AAA AAA AAA AAA AAA AAA AAA AAA

(2) INFORMATION FOR SEQ ID NO:2:
(i) SEQUENCE:
(A) LENGTH: 99 AMINO ACID
(B) TYPE: AMINO ACID
(C) STRANDEDNESS: SINGLE
(D) TOPOLOGY: LINER
(ii) MOLECULE TYPE: PROTEIN
(iii) SEQUENCE DESCRIPTION: SEQ ID NO: 2

5 10 15
| | |
1 Met Asp Asn Val Gln Pro Lys Ile Lys His Arg Pro Phe Cys Phe
16 Ser Val Lys Gly His Val Lys Met Leu Arg Leu Asp Ile Ile Asn
31 Ser Leu Val Thr Thr Val Phe Met Leu Ile Val Ser Val Leu Ala
46 Leu Ile Pro Glu Thr Thr Thr Leu Thr Val Gly Gly Gly Val Phe
61 Ala Leu Val Thr Ala Val Cys Cys Leu Ala Asp Gly Ala Leu Ile
76 Tyr Arg Lys Leu Leu Phe Asn Pro Ser Gly Pro Tyr Gln Lys Lys
91 Pro Val His Glu Lys Lys Glu Val Leu

(1) INFORMATION FOR SEQ ID NO: 3:
i) SEQUENCE
(A) LENGTH: 459 BASE PAIRS
(B) TYPE: NUCLEIC ACID
(C) STRANDEDNESS: SINGLE
(D) TOPOLOGY: LINEAR
(ii) MOLECULE TYPE: cDNA
(iii) SEQUENCE DESCRIPTION: SEQ ID NO:3

3 9 15 21 27 33 39 45
| | | | | | | |
1 ATG GAT AAC GTG CAG CCG AAA ATA AAA CAT CGC CCC TTC TGC TTC
46 AGT GTG AAA GGC CAC GTG AAG ATG CTG CGG CTG GCA CTA ACT GTG
91 ACA TCT ATG ACC TTT TTT ATC ATC GCA CAA GCC CCT GAA CCA TAT
136 ATT GTT ATC ACT GGA TTT GAA GTC ACC GTT ATC TTA TTT TTC ATA
181 CTT TTA TAT GTA CTC AGA CTT GAT CGA TTA ATG AAG TGG TTA TTT
226 TGG CCT TTG CTT GAT ATT ATC AAC TCA CTG GTA ACA ACA GTA TTC
271 ATG CTC ATC GTA TCT GTG TTG GCA CTG ATA CCA GAA ACC ACA ACA
316 TTG ACA GTT GGT GGA GGG GTG TTT GCA CTT GTG ACA GCA GTA TGC
361 TGT CTT GCC GAC GGG GCC CTT ATT TAC CGG AAG CTT CTG TTC AAT
406 CCC AGC GGT CCT TAC CAG AAA AAG CCT GTG CAT GAA AAA AAA GAA
451 GTT TTG TAA

(2) INFORMATION FOR SEQ ID NO:4:
(i) SEQUENCE:
(A) LENGTH: 152 AMINO ACID
(B) TYPE: AMINO ACID
(C) STRANDEDNESS: SINGLE
(D) TOPOLOGY: LINER
(ii) MOLECULE TYPE: PROTEIN
(iii) SEQUENCE DESCRIPTION: SEQ ID NO: 4

5 10 15
| | |
1 Met Asp Asn Val Gln Pro Lys Ile Lys His Arg Pro Phe Cys Phe
16 Ser Val Lys Gly His Val Lys Met Leu Arg Leu Ala Leu Thr Val
31 Thr Ser Met Thr Phe Phe Ile Ile Ala Gln Ala Pro Glu Pro Tyr
46 Ile Val Ile Thr Gly Phe Glu Val Thr Val Ile Leu Phe Phe Ile
61 Leu Leu Tyr Val Leu Arg Leu Asp Arg Leu Met Lys Trp Leu Phe
76 Trp Pro Leu Leu Asp Ile Ile Asn Ser Leu Val Thr Thr Val Phe
91 Met Leu Ile Val Ser Val Leu Ala Leu Ile Pro Glu Thr Thr Thr
106 Leu Thr Val Gly Gly Gly Val Phe Ala Leu Val Thr Ala Val Cys
121 Cys Leu Ala Asp Gly Ala Leu Ile Tyr Arg Lys Leu Leu Phe Asn
136 Pro Ser Gly Pro Tyr Gln Lys Lys Pro Val His Glu Lys Lys Glu
151 Val Leu

(1) INFORMATION FOR SEQ ID NO: 5:
(i) SEQUENCE:
(A) LENGTH: 204 BASE PAIRS
(B) TYPE: NUCLEIC ACID
(C) STRANDEDNESS: SINGLE
(D) TOPOLOGY: LINEAR
(ii) MOLECULE TYPE: cDNA
(iii) SEQUENCE DESCRIPTION: SEQ ID NO: 5

3 9 15 21 27 33 39 45
| | | | | | | |
1 ATG GAT AAC GTG CAG CCG AAA ATA AAA CAT CGC CCC TTC TGC TTC
46 AGT GTG AAA GGC CAC GTG AAG ATG CTG CGG CTG GTG TTT GCA CTT
91 GTG ACA GCA GTA TGC TGT CTT GCC GAC GGG GCC CTT ATT TAC CGG
136 AAG CTT CTG TTC AAT CCC AGC GGT CCT TAC CAG AAA AAG CCT GTG
181 CAT GAA AAA AAA GAA GTT TTG TAA

(2) INFORMATION FOR SEQ ID NO:6:
(i) SEQUENCE:
(A) LENGTH: 67 AMINO ACID
(B) TYPE: AMINO ACID
(C) STRANDEDNESS: SINGLE
(D) TOPOLOGY: LINER
(ii) MOLECULE TYPE: PROTEIN
(iii) SEQUENCE DESCRIPTION: SEQ ID NO: 6

5 10 15
| | |
1 Met Asp Asn Val Gln Pro Lys Ile Lys His Arg Pro Phe Cys Phe
16 Ser Val Lys Gly His Val Lys Met Leu Arg Leu Val Phe Ala Leu
31 Val Thr Ala Val Cys Cys Leu Ala Asp Gly Ala Leu Ile Tyr Arg
46 Lys Leu Leu Phe Asn Pro Ser Gly Pro Tyr Gln Lys Lys Pro Val
61 His Glu Lys Lys Glu Val Leu

(1) INFORMATION FOR SEQ ID NO: 7:
( i) SEQUENCE:
(A) LENGTH: 363 BASE PAIRS
(B) TYPE: NUCLEIC ACID
(C) STRANDEDNESS: SINGLE
(D) TOPOLOGY: LINEAR
(ii) MOLECULE TYPE: cDNA
(iii) SEQUENCE DESCRIPTION: SEQ ID NO: 7

3 9 15 21 27 33 39 45
| | | | | | | |
1 ATG GAT AAC GTG CAG CCG AAA ATA AAA CAT CGC CCC TTC TGC TTC
46 AGT GTG AAA GGC CAC GTG AAG ATG CTG CGG CTG GCA CTA ACT GTG
91 ACA TCT ATG ACC TTT TTT ATC ATC GCA CAA GCC CCT GAA CCA TAT
136 ATT GTT ATC ACT GGA TTT GAA GTC ACC GTT ATC TTA TTT TTC ATA
181 CTT TTA TAT GTA CTC AGA CTT GAT CGA TTA ATG AAG TGG TTA TTT
226 TGG CCT TTG CTT GTG TTT GCA CTT GTG ACA GCA GTA TGC TGT CTT
271 GCC GAC GGG GCC CTT ATT TAC CGG AAG CTT CTG TTC AAT CCC AGC
316 GGT CCT TAC CAG AAA AAG CCT GTG CAT GAA AAA AAA GAA GTT TTG
361 TAA

(2) INFORMATION FOR SEQ ID NO:8:
(i) SEQUENCE:
(A) LENGTH: 120 AMINO ACID
(B) TYPE: AMINO ACID
(C) STRANDEDNESS: SINGLE
(D) TOPOLOGY: LINER
(ii) MOLECULE TYPE: PROTEIN
(iii) SEQUENCE DESCRIPTION: SEQ ID NO: 8

5 10 15
| | |
1 Met Asp Asn Val Gln Pro Lys Ile Lys His Arg Pro Phe Cys Phe
16 Ser Val Lys Gly His Val Lys Met Leu Arg Leu Ala Leu Thr Val
31 Thr Ser Met Thr Phe Phe Ile Ile Ala Gln Ala Pro Glu Pro Tyr
46 Ile Val Ile Thr Gly Phe Glu Val Thr Val Ile Leu Phe Phe Ile
61 Leu Leu Tyr Val Leu Arg Leu Asp Arg Leu Met Lys Trp Leu Phe
76 Trp Pro Leu Leu Val Phe Ala Leu Val Thr Ala Val Cys Cys Leu
91 Ala Asp Gly Ala Leu Ile Tyr Arg Lys Leu Leu Phe Asn Pro Ser
106 Gly Pro Tyr Gln Lys Lys Pro Val His Glu Lys Lys Glu Val Leu

## Description

### FIELD OF THE INVENTION

The present invention provides a novel chemokine-like factor 1 (CKLF1) polypeptide and variants thereof, which have been identified as having chemotactic and hematopoietic stimulating activities. Also provided are polynucleotides encoding the CKLF1 polypeptide and variants thereof, and a method to produce such polypeptides. The present invention further discloses antibodies and antagonists of such CKLF polypeptides. Also disclosed are uses of such CKLF polynucleotides and polypeptides in the diagnosis and treatment of diseases of immune system, diseases of hematopoietic system and tumors.

### BACKGROUND OF THE INVENTION

Cytokines refer to small polypeptides synthesized and secreted by organic cells, which participate in the proliferation and differentiation of many types of cells and play important roles in physiological and pathological processes. Cytokines include such members as interleukins, colony stimulating factors, interferons, tumor necrosis factors, growth factors and chemokines *etc.* Some drugs containing recombinant cytokines or cytokine antagonists produced by bioengineering techniques have been applied to clinical treatment and have enjoyed positive effects, which demonstrates the spacious applications of cytokines.

Chemokines (also called chemotactic factors) are dozens of small polypeptides with similar structure and average molecular weight between 8-12 KD, These polypeptides have chemotactic effects and play important roles in immune defense, immunoregulation, inflammation, hematopoietic regulation and vasculogenesis. The amino acid sequences of most of chemokines share the same character of the conserved four cysteine (Cys) motif. According to the positions of the first and the second Cys, chemokines can be divided into four subfamilies named CXC, C-C, CX₃C and C, respectively, wherein C represents Cys and X represents any kind of amino acid. The members of CXC, for example, interleukin-8 (IL-8), interferon-induced protein 10 (IFN-IP-10) and cytokine with melanocyte growth stimulating activity (MGSA), have their encoding genes usually located on chromosome 4 in human somatic cells, and activate and chemotacte neutrophils and T lymphocytes. While the numbers of C-C, such as macrophage inflammatory protein-1α (MIP-1α), macrophage inflammatory protein-1β (MIP-1β), monocyte chemoattractant protein-1(MCP-1), and cytokine regulated upon activation, normal T cell expressed and presumably secreted (RANTES), have their encoding genes usually located on chromosome 17 in human somatic cells, and mainly activate and chemotacte monocytes, lymphocytes, basophils and eosinophils. Fractalkine is the only identified number of the CX₃C subfamily, which can activate and chemotacte T cells and monocytes. As for the C subfamily, only Lymphotactin (Ltn) is presently found, which can chemotacte lymphocytes.

The receptors of chemokines belong to the superfamily of GTP-binding proteins that have a character of seven transmembrane domains. In accordance with their combination to different chemokines, chemokine receptors are named CXC Receptor (CXCR), such as CXCR1, CXCR2, CXCR3, and CXCR4; C-C Receptor (CCR), such as CCR1, CCR2, CCR3, CCR4, CCR5, CCR6 and CCR7; CX₃C Receptor (CX₃CR) (See Marco, B., *et al.*, Human Chemokines: *An Update Annu. Rev. Immunol*. 1997, 15:675-705).

Due to the significant roles chemokines perform in systematic immune, inflammatory responses and hematopoietic regulations, some abnormal changes of chemokines and chemokine receptors *(e.g.,* defects of chemokines or chemokine receptors, or overexpression of chemokines, or increased level of soluble chemokine receptors) always result in infectious diseases or autoimmune diseases, or sometimes even in tumors. Therefore, the detection of chemokines or chemokine receptors may be used in clinical diagnosis to observe the development of diseases and to judge the curative effects. Recent studies show that HIV can invade immunocytes of human by combining with chemokine receptors, which indicate that chemokines are closely related to the infectious and pathological courses of HIV. Hence chemokines may become promising drugs to prevent HIV from invading immune cells (Cocchi, F., *et al.*, "Identification of RANTES, MIP-1α, and MIP-1β as the Major HIV-Suppressive Factors Produced by CD8+ T Cells," *Science,* 1995, 270:1811). Furthermore, chemokines produced by bioengineering techniques and drugs containing such chemokines are expected to be novel bioengineering medicines in the future. Some bioengineering medicines containing chemokines have been applied to clinical phase in succession, for instance, myeloid progenitor inhibitory factor-1 (MPIF-1) has been used as a protective hematopoietic drug in high-dose radiotherapy or chemotherapy for tumors (Marshall, A., "HGS launches "first" genomics product in clinic," *Nat. Biotechol.* 1998, 16:129).

The present invention is based on the ideas that many cytokines or chemokines can be stimulated by phytohemagglutinin (PHA) (Brantschen, S., *et al.*, " Differential expression of cytokine mRNAs cell lines," *Lymphokine Res.* 1989, 8 (3):163-72), while interleukin-10 (IL-10) is a broad-spectrum inhibitor of cytokines (Di-Hwei, H., *et al.*, *Science,* 1990, 250:830). By using suppression subtractive hybridization (SSH) method (Diatchenko, L., *et al*, "Suppression subtractive hybridization: A method for generating differentially regulated or tissue-specific cDNA probes and libraries," *Pro. Natl. Acad. Sci. USA*. 1996, 93:6025-30), some unknown cytokines may be found. Based on the above, the present inventors, by using SSH method, have identified and cloned CKLF1 gene from a cDNA library which is derived from U937, a human promonocytic cell line. The inventors further disclose the biological activity and function of CKLF1 and variants thereof.

### SUMMARY OF THE INVENTION

The first aspect of the present invention provides a chemokine-like factor 1 (CKLF1) polypeptide and variants, analogs, derivatives and fragments thereof, which are identified as having chemotactic activities on leucocytes and stimulating effects on hematopoietic progenitor cells.

The second aspect of the present invention provides polynucleotides (nucleoid acid moleculars) encoding such CKLF1 polypeptide and variants, analogs and derivatives thereof. The said polynucleotides include DNAs, cDNAs, genomic DNAs and mRNAs.

The third aspect of the present invention provides a method of producing such CKLF polypeptides by recombinant techniques comprising cloning polynucleotides encoding such CKLF polypeptides into suitable vectors, introducing recombinant vectors into prokaryotic and/or eukaryotic host cells by transduction, transfection or transformation *etc.,* and obtaining the expressed product from host cells (*e.g.* from cell lysate and/or from extracellular media).

The fourth aspect of the present invention provides a method of employing such CKLF polypeptides and/or their coding polynucleotides to make medical compositions used in the diagnosis and treatment of such diseases as primary tumors, inferior hematopoiesis and diseases caused by functionally abnormal immunocytes.

The fifth aspect of the present invention provides a medical composition comprising mixing CKLF polypeptides or their biologically active fragments with one or more pharmaceutically acceptable carrier(s) and/or excipient(s).

The sixth aspect of the present invention provides polyclonal or monoclonal antibodies specific to such CKLF polypeptides.

The seventh aspect of the present invention provides antagonists of the present polypeptides, which may be used to inhibit the action of CKLF polypeptides. It further provides a method of using such antagonists in the treatment of rheumatoid arthritis, autoimmune diseases, tumors and diseases caused by virus infection.

The following drawings and examples are given to illustrate embodiments of the present invention. They are merely exemplary and are not intended to limit the scope of the invention otherwise described herein.

### BRIEF DESCRIPTION OF DRAWINGS AND SEQUENCE LISTING

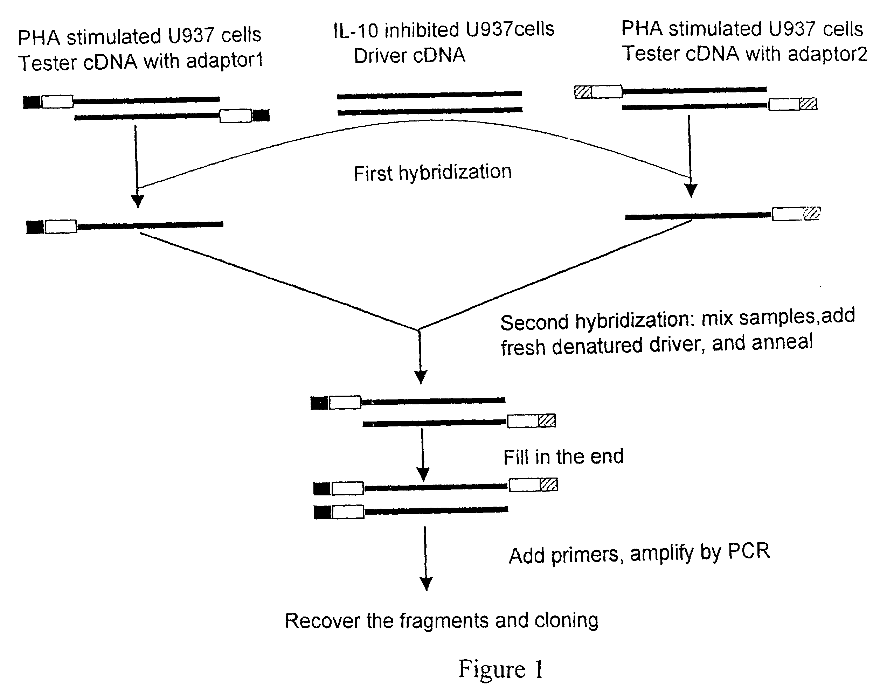

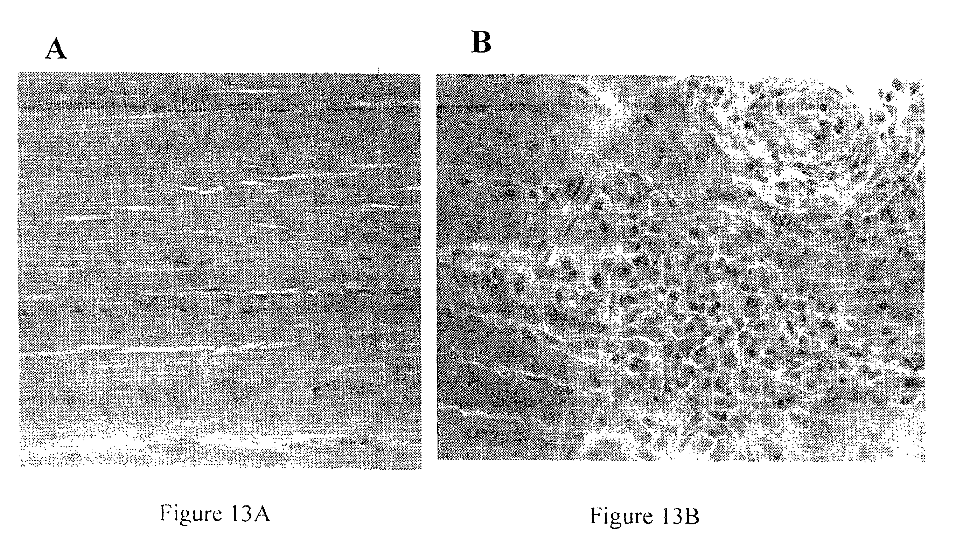

Figure1 depicts the process of isolating cDNAs from human U937 cells by suppression subtractive hybridization (SSH) method. Driver cDNA comes from U937 cells inhibited with IL-10, tester cDNA comes from U937 cells stimulated with PHA.
Figure 2 shows the structural differences of the CKLF1, 2, 3 and 4 polypeptides
Figure 3 shows the expression of CKLF1 mRNA in U937 cells by Northern blot.
Figure 4 is the result of SDS-PAGE stained with Coomassie blue, which shows the expression of recombinant plasmid pMTY4-CKLF1 in *Escherichia coli.*
Figure 5A -5I indicate chemotaxis of CKLF1 to different eukaryotic cells.

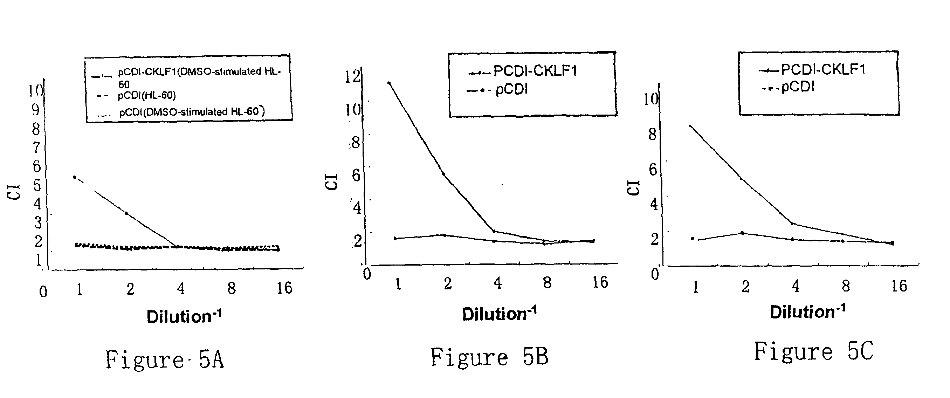

Figure 5A shows chemotaxis of supernatants of pcDI-CKLF1-transfected COS-7 cells to the DMSO stimulated HL-60 cells.
Figure 5B shows chemotaxis of supernatants of pcDI-CKLF1-transfected COS-7 cells to mouse celiac macrophages.
Figure 5C shows chemotaxis of supernatants of pcDI-CKLF1-transfected COS-7 cells to mouse lymphocytes.

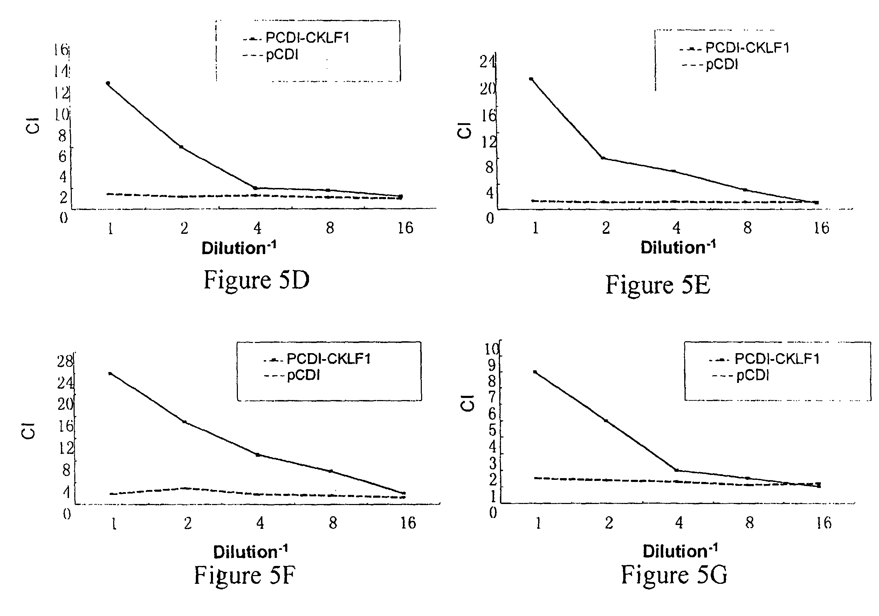

Figure 5D shows chemotaxis of supernatants of pcDI-CKLF1-transfected COS-7 cells to human PBMCs.
Figure 5E shows chemotaxis of supernatants of pcDI-CKLF1-transfected COS-7 cells to human neutrophils.
Figure 5F shows chemotaxis of supernatants of pcDI-CKLF1-transfected COS-7 cells to U937 cells.
Figure 5G shows chemotaxis of supernatants of pcDI-CKLF1-transfected COS-7 cells to K526 cells.

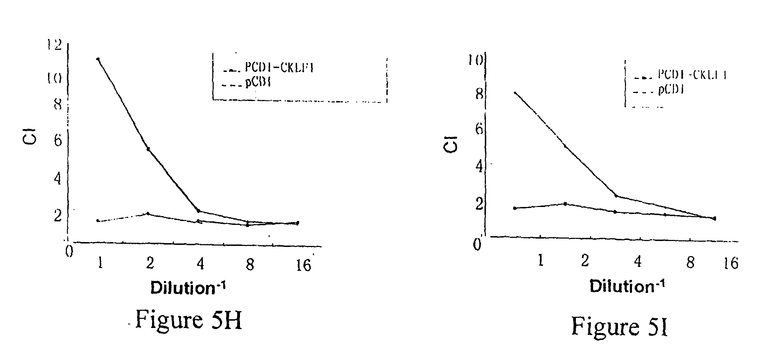

Figures 5H and 5I show chemotaxis of supernatants of pcDI-CKLF1-transfected COS-7 cells to mouse celiac macrophages and lymphocytes, respectively.

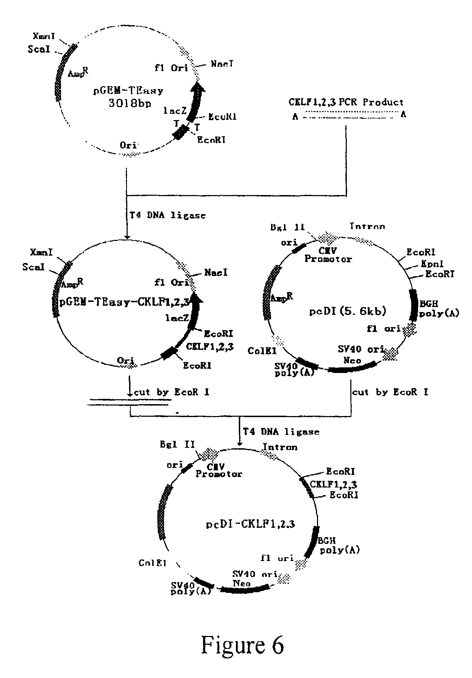

Figure 6 depicts the process of constructing a eukaryotic express vector pcDI-CKLF1. Construction of plasmids pcDI-CKLF2, 3 and 4 is similar to that of pcDI-CKLF1.

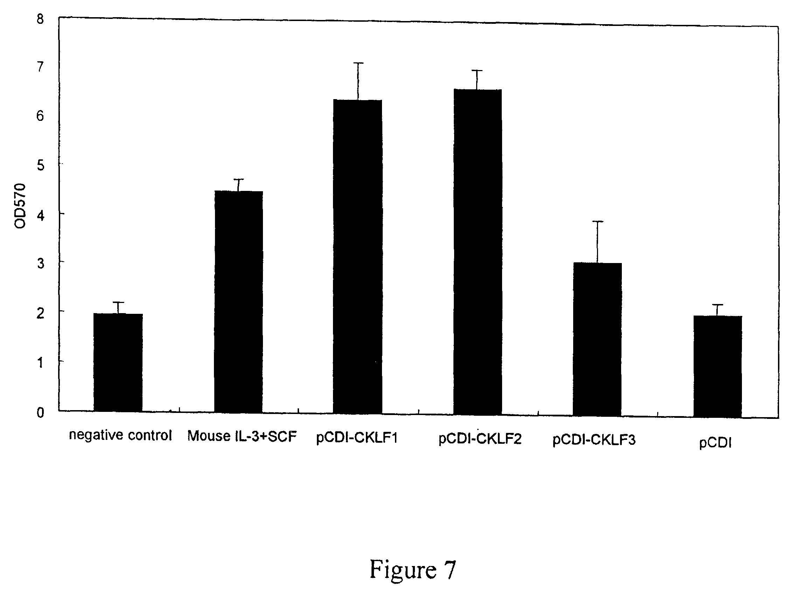

Figure 7 depicts proliferation effects of the recombinant plasmids pcDI-CKLF1, 2 and 3 to mouse bone marrow cells.

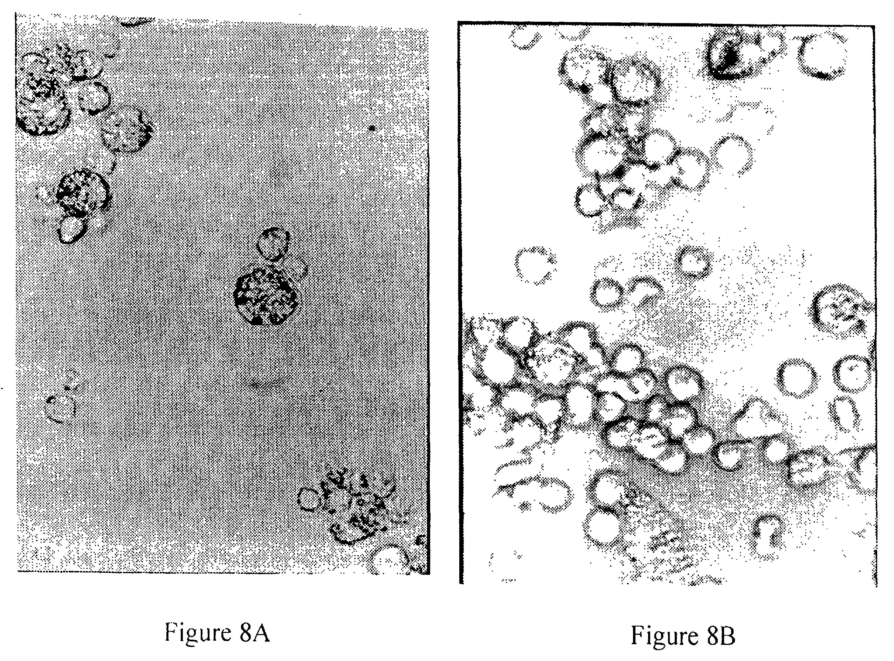

Figures 8A and 8B show the shape change of mouse bone marrow cells after stimulation with CKLF1. Figure 8A shows the result of the pcDI group. Figure 8B shows the result of the pcDI-CKLF I group.

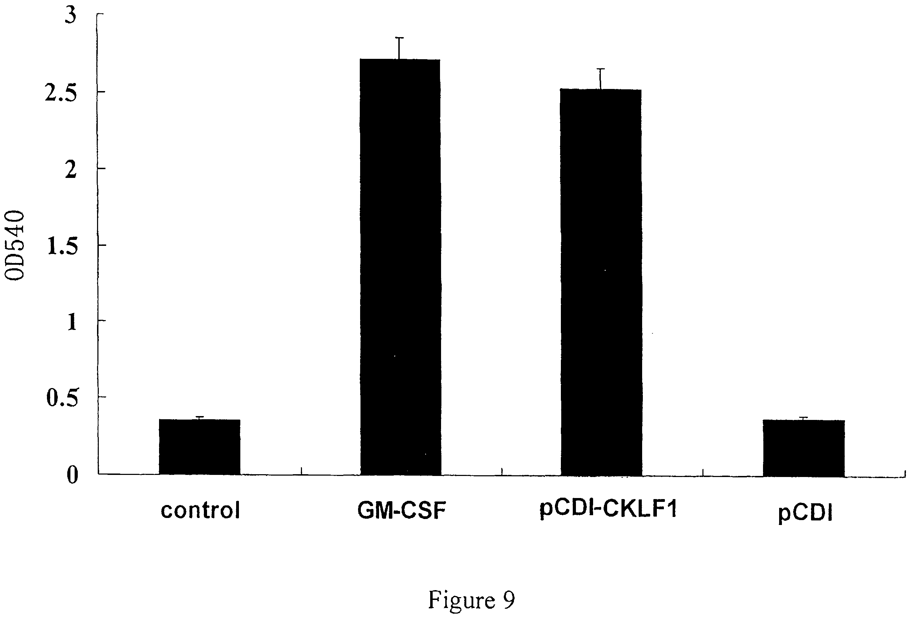

Figure 9 is the result of MTT test, which indicates promoting effect of supernatants of pcDI-CKLF1-transfected COS-7 cells to the proliferation of human bone marrow cells.

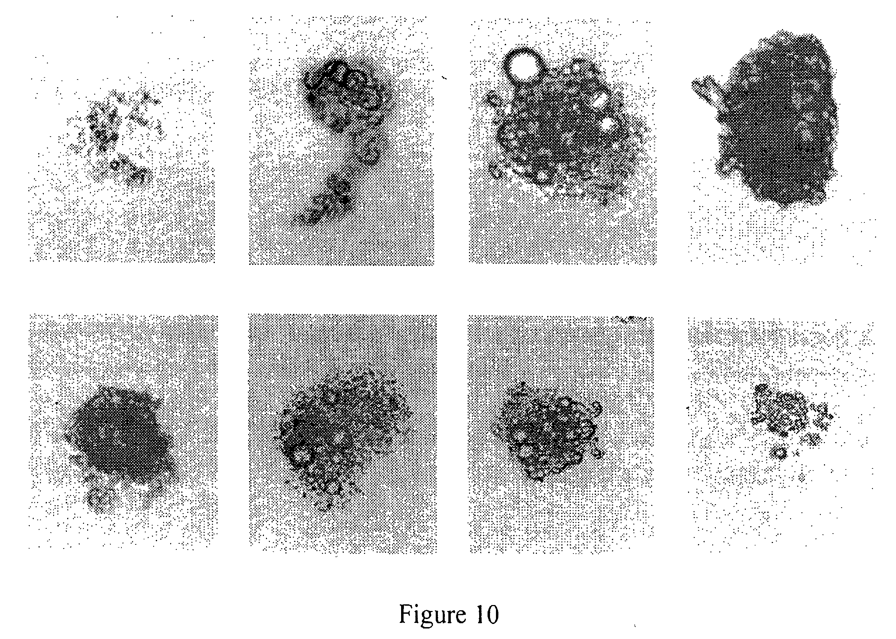

Figure 10 shows colonial morphology of human bone marrow cells after stimulation with supernatants of pcDI-CKLF1-transfected COS-7 cells (incubated for 20 days).

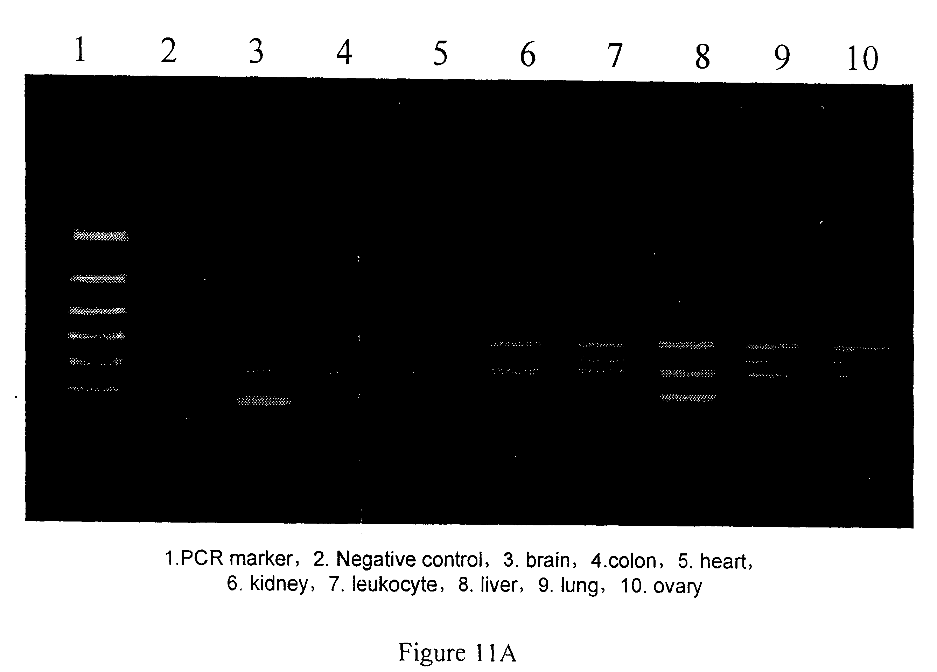

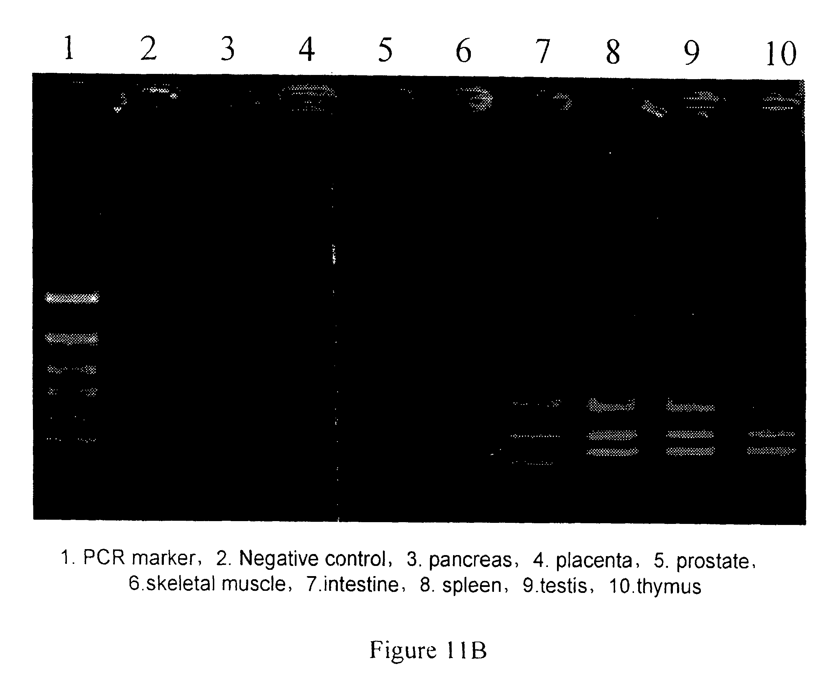

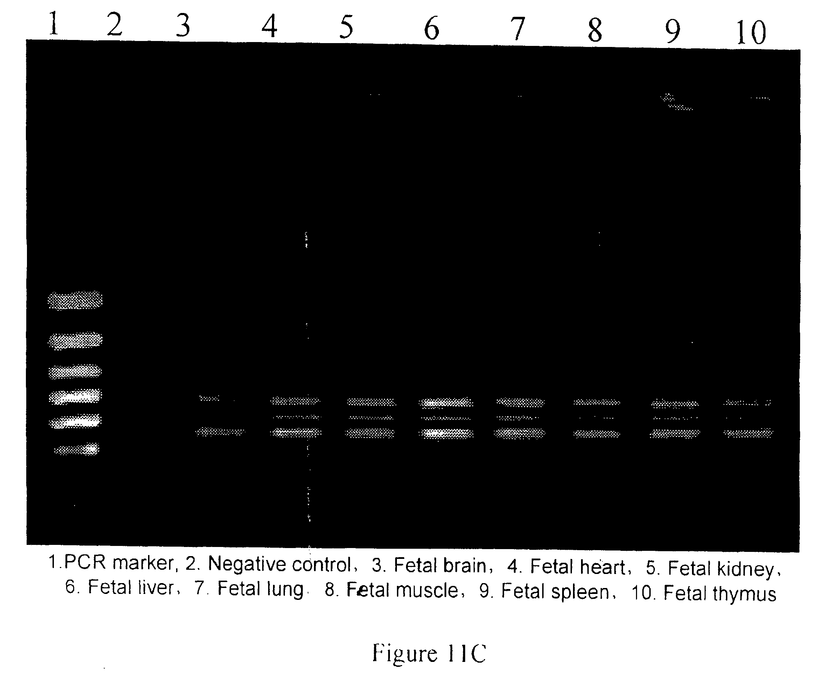

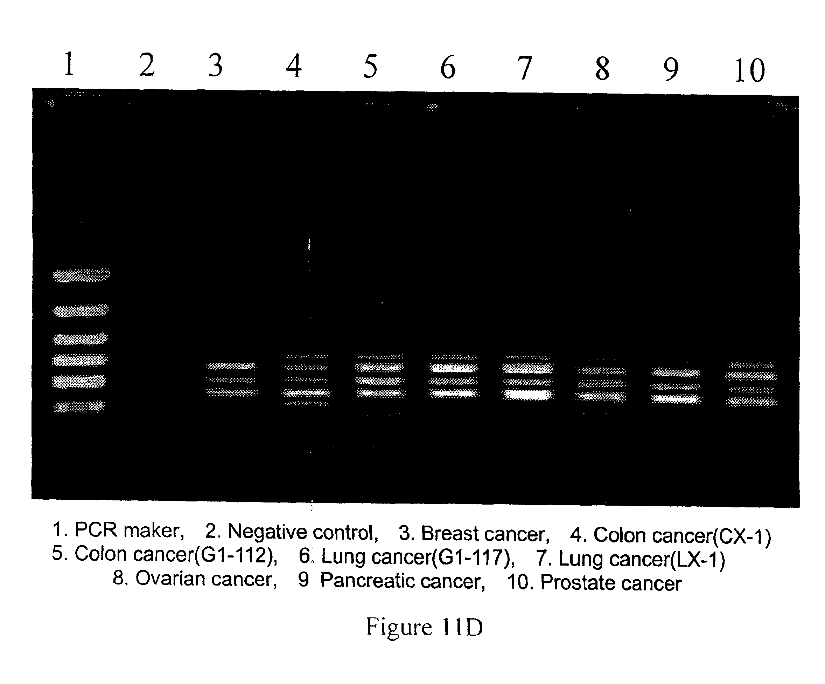

Figures 11A and 11B show expression of CKLF1 and its variants in tissues of healthy adult. Figures 11C and 11D display expression of CKLF1 and its variants in fetal tissues and many tumor tissues.

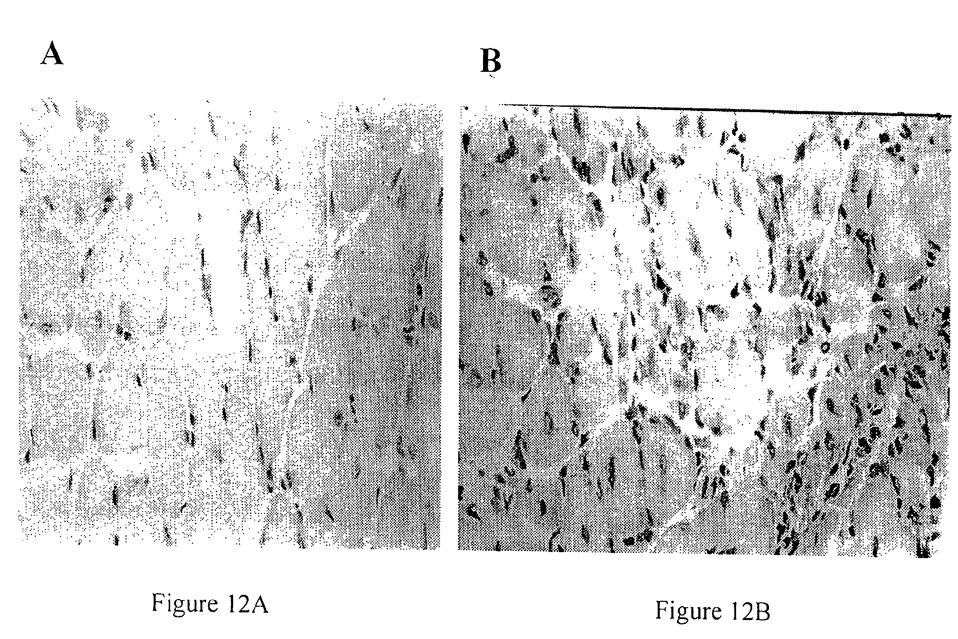

Figures 12A and 12B show changes of cross-sections of anterior tibial muscle in BALB/c mice after direct injection of pcDI and pcDI-CKLF1 plasmids, respectively.
   Cross-sections were stained with Acid Phosphatase (AcP) and Hematoxylin-Eoxin (HE) 10 days after injection. Figure 12A shows the result of the pcDI control group, while Figure 12B shows the result of the pcDI-CKLF1 sample group, in which muscle fiber regeneration was evidently observed. Original magnification: 40 ×
Figures 13A and 13B show changes of vertical-sections of anterior tibial muscle in BALB/c mice after directly injection of pcDI and pcDI-CKLF1. Vertical-sections were stained with HE 10 days after injection. Figure 13A shows the result of the pcDI control group, while figure 13B shows the result of the pcDI-CKLF1 sample group, in which muscle fiber regeneration was evidently observed. Original magnification: 40 ×
SEQ ID NO: 1 displays the polynucleotide of CKLF1, including the open reading frame of CKLF1 cDNA.
SEQ ID NO: 2 displays the amino acid sequence of CKLF1 deduced from SEQ ID NO: 1.
SEQ ID NO: 3 displays the polynucleotide of CKLF2.
SEQ ID NO: 4 displays the amino acid sequence of CKLF2 deduced from SEQ ID NO: 3.
SEQ ID NO: 5 displays the polynucleotide of CKLF3.
SEQ ID NO: 6 displays the amino acid sequence of CKLF3 deduced from SEQ ID NO: 5.
SEQ ID NO: 7 displays the polynucleotide of CKLF4.
SEQ ID NO: 8 displays the amino acid sequence of CKLF4 deduced from SEQ ID NO: 7.

### DETAILED DESCRIPTION OF THE INVENTION

One aspect of the present invention provides a chemokine-like factor 1(CKLF1) polypeptide with chemotactic activity on immune cells and stimulatory effect on hematopoietic progenitor cells. The CKLF1 polypeptide, encoded by the polynucleotide as shown in SEQ ID NO: 1, has the amino acid sequence as set forth in SEQ ID NO: 2 or that expressed by the recombinant plasmid contained in CGMCC Deposit NO.0392. Chemokine-like factor (CKLF) is originally called U937-derived chemokine (UCK), and then changed to the present appellation with the kind suggestion of International Committee on Standardized Genetic Nomenclature.

The CKLF1 polypeptide is composed of 99 amino acids with molecular weight of 10,923 Dalton. It has been shown that two successive cysteine residues, a typical structural character of the C-C chemokine subfamily, are contained in the CKLF1 polypeptide, and that no typical domainsignal peptide cleavage site, DNA biding site and putative N-glycosylation site have been found in the deduced CKLF1 polypeptide. The amino acids from residue 12 through residue 20 at the N-terminal are hydrophobic and may contain a natural cleavage site. It has also found that the CKLF1 polypeptide shares no obvious homology with any known protein, but shares 46% homology with permease of *Caenorhabditis elegan* from residue 35 through residue 79. The CKLF1 polypeptide has been identified as a secretory one, which can be detected in many tumor tissues and human embryo tissues, such as colon, pancreas, brain and heart. It has been further observed that the CKLF1 polypeptide shows remarkable chemotactic activity to some leucocytes and promoting activity to the proliferation of hematopoietic cells and skeletal cells (See Examples 6, 7, 8 and 10).

The present invention also relates to variants of the CKLF1 polypeptide, which may include, but not limit to, the identified CKLF2, CKLF3 and CKLF4 polypeptides as set forth in SEQ ID NO: 4, SEQ ID NO: 6 and SEQ ID NO: 8, respectively. The polynucleotides encoding such CKLF2, CKLF3 and CKLF4 polypeptides have been shown in SEQ ID NO: 3, SEQ ID NO: 5 and SEQ ID NO: 7, respectively. The deduced amino acid sequences of the CKLF2, CKLF3 and CKLF 4 polypeptides have 152, 67 and 120 residues, respectively. It has been assumed that variants are formed by differently splicing the CKLF1 gene during the post-transcription course. The expression of the CKLF2, CKLF3 and CKLF4 polypeptides have also been found in embryo tissues and tumor tissues such as those from colon cancer, lung cancer, prostate cancer and ovarian cancer. In particular, the expression level of CKLF2 is low in normal cells, but much higher in embryonic cells and primary tumor cells.

The present invention further relates to analogs, derivatives and fragments of the said CKLF1 polypeptide. "Analog" herein used means polypeptides having at least 70%, preferably at least 90% and more preferably at least 95% homology (preferably identity) to the CKLF1 polypeptide described above. As known in the art, " homology" between two polypeptides is determined by comparing the amino acid sequence and the conserved amino acid substitutes of one polypeptide to the sequence of a second polypeptide. The derivative of the CKLF1 polypeptide may be: (i) one in which one or more of the amino acid residuesa includes a substituent group; or (ii) one in which the mature polypeptide is fused with the additional amino acids, such as additional amino acids in one terminal of the CKLF1 polypeptide for better purification; or (iii) one in which the mature polypeptide is fused with a compound, such as a compound containing polyethylene glycol or lipid to increase the half-life of the polypeptide. The fragment of the CKLF1 polypeptide means a polypeptide with amino acid sequence partially or totally identical to the sequence as set forth in SEQ ID NO: 2, such fragment is composed of at least 20, preferably more than 40 amino acid residues. More preferably, such fragment has identical or similar biological activity or function to that of the CKLF1 polypeptide.

The polypeptide of the present invention may be a natural polypeptide, a synthetic polypeptide, a recombinant polypeptide produced from prokaryotic or eukaryotic cells by DNA recombinant techniques or produced from cell-free translation systems by using mRNA derived from the CKLF polynucleotide of the present invention. A recombinant polypeptide is more preferable. In the process of peptide synthesis, a fragment of the CKLF1 polypeptide may be employed as an intermediate to produce the full-length polypeptide. While in the process of producing a polypeptide by recombinant techniques, many cells may be selected as host cells. Hence the polypeptide of the present invention may be glycosylated or non-glycosylated, and may contain a methionine as the initial amino acid residue.

The CKLF polypeptides of the present invention are preferably provided in an isolated form, and more preferably are purified to homogeneity. "Isolated" used herein means that the material is removed from its original environment (e.g. the living organism or the natural environment if it is naturally occurring).

The second aspect of the present invention provides the polynucleotide encoding the CKLF1 polypeptide. As shown in SEQ ID NO: 1, The full length cDNA of the CKLF1 polynucleotide has 552 base pairs including a "ATTAAA" polyadenylation signal and a polyadenylic acid (poly(A)) tail, wherein the open reading frame is from nucleotide 152 to nucleotide 449. The CKLF1 polynucleotide, with the GenBank accession number AF096895, exhibits little homology (identity) to known genes.

The polynucleotide of the present invention may be in the form of RNA or in the form of DNA, which DNA includes cDNA, genomic DNA, and synthetic DNA. The DNA may be double-stranded or single-stranded, and if single stranded it may be the coding strand or non-coding (anti-sense) strand. The polynucleotide of the present invention is preferably provided in an isolated form. Such polynucleotide may include: (i) only the coding sequence for the mature polypeptide; (ii) the coding sequence for the mature polypeptide and the additional coding sequence; (iii) the coding sequence for the mature polypeptide and non-coding sequence, such as introns or non-coding sequences at the 5' and/or 3' terminal(s) of the coding sequence. The coding sequence which encodes the mature CKLF1 polypeptide may be identical to the coding sequence as set forth in SEQ ID NO: 1 or to that in the deposited clone CGMCC NO.0392 or may be a different coding sequence which, as a result of the redundancy or degeneracy of the genetic code, encodes the same mature polypeptide as the DNA of SEQ ID NO: 1 or the deposited cDNA.

A fragment of CKLF1 polynucleotide may be used as a probe in a cDNA library to isolate or detect the full length CKLF1 polynucleotide, or to recover polynucleotides sharing high sequence homology with the CKLF1 gene. Probes may contain at least 30 bases and preferably more than 50 bases. Sequences capable of hybridizing to the labeled probe under suitable conditions may be isolated from a cDNA library, a genomic DNA library or a mRNA library. Such sequences contain at least 10 bases and preferably more than 30 bases.

The present invention further relates to variants of the CKLF1 polynucleotide. more specifically, the polynucleotides of CKLF2, CKLF3 and CKLF4 as set forth in SEQ ID NO: 3, SEQ ID NO: 5 and SEQ ID NO: 7, respectively. Compared with the coding sequence of the CKLF1 polynucleotide, no frame shift mutation is observed in that of the CKLF2, CKLF3 and CKLF4 polynucleotides which are speculated to be allelic gene variants of SEQ ID NO: 1. The polynucleotides of CKLF2, CKLF3 and CKLF4 can be searched in GenBank database published after October 1999, the accession numbers of which are AF135380, AF135381, AF145216, respectively.

The present invention further relates to polynucleotides which hybridize to the above-described CKLF1 gene if there is at least 70%, preferably at least 90%, and more preferably at least 95% homology between the sequences, particularly relates to polynucleotides which hybridize under stringent conditions to the above-described CKLF1 polynucleotide. "Stringent conditions" herein used means hybridization will occur only if there is at least 95% homology between the sequences. Such polynucleotide may be a naturally occurring or a non-naturally occurring one. As indicated above, such polynucleotide may be an allelic variant of the CKLF1 polynucleotide, or may include a deletion, addition or substitution of one or more nucleotides in the CKLF1 polynucleotide. The polypeptide encoded by such polynucleotide may have or have not the same function as the mature CKLF1 polypeptide, preferably substantially the same biological activity or function.

The third aspect of the present invention provides a method to produce the CKLF1 polypeptide by recombinant techniques comprising constructing a vector containing the CKLF1 polynucleotide₂ introducing such a vector into a host cell, and obtaining the CKLF polypeptide from the said host cell.

The CKLF polynucleotide may be inserted into one or more appropriate restriction endonuclease site(s) in a vector. Vectors may be chromosomal derived, nonchromosomal derived, or synthetic vectors, for example, they may be derivatives of SV40 phage, bacterial plasmids, phage DNA, yeast plasmids, phagemids (vectors derived from the combination of plasmids and phages) and virus vectors (e.g. baculovirus, vaccine virus, adenovirus and fowl pox virus). Generally, the vectors will include such genetic components as origins of replication, promoters and selectable marker genes. Examples of such promoters are: the respiratory syncytial virus (RSV) promoter, the human immunodeficiency virus (HIV) promotor, the cytomegalovirous (CMV) promoter, the SV40 promoter, the *Escherichia coli* (*E. coli*) *lac* or *trp* promoters and the phage lambda promoter (λP_{L}). Examples of selectable marker genes include neomycin resistance gene and dihydrofolate reductase gene suitable for selecting from transformed eukaryotic cells, or ampicillin resistance gene and tetracycline gene suitable for selecting from transformed *E. coli* cells. The expression vectors may also contain transcription terminators and ribosome binding sites (RBS) to initiate the translation. The vectors used for expressing polypeptide from eukaryotic cells generally contain such genetic regulatory components as eukaryotic promoters (e.g. CMV immediate early promoter, SV40 promoter, HSV thymidine kinase promoter) and enhencers. Enhencers are cis-acting elements of DNA, usually from about 10 to 300 base pairs that act on a promoter to increase its transcription. A leader sequence capable of directing secretion of translated protein into the periplasmic space or extracellular medium may be inserted into the vector between the promoter sequence and the structural DNA sequence if necessary. Such vectors suitable for expression from eukaryotic cells include pMT-hIL3 (Ma Dalong, *et al*., 1991, High-tech Commumication 11: 26-29), pQE-9 (Qiagen), pD10 and Pnh18A (Stratagene), pKK233-3, pDR540 and pRIT5 (Pharmacia), pcDNA3 (Invitrogen), pCI (Promega), pWLNEO and pSG (Stratagene), pSVL (Pharmacia), *etc.*

Vectors containing the CKLF1 polynucleotide of the present invention can be transfered into host cells by the method of transduction, transformation or transfection. As known in the art, the conventional methods includes calcium phosphate transfection, diethyl aminoethyl-dextran (DEAE-Dextran) mediated transfection, or electroporation. Mature polypeptides can be expressed in suitable host cells. Representative examples of such host cells are: (i) bacterial cells, such as *Escherichia coli, Salmonella typhimurium* and *Streptomyces;* (ii) fungal cells, such as yeast; (iii) insect cells, such as Drosophila S2 and SDodoptera Sf9; (iv) mammalian cells, such as CHO, COS and HEK293; (v) human derived cells, such as TF-1, U937 and Hela, etc. In the preferred embodiments of the present invention, higher eukaryotic cells, such as normal and tumor cells from mammalian lower eukaryotic cells, such as yeast cells and prokaryotic cells, such as cells from *Escherichia coli*, were employed as hosts.

Host cells can be cultured in conventional nutrient media or special media. Special media herein used mean modified nutrient media as appropriate for activating promoters, screening transformants or amplifying genes. As known in the art, the culturing conditions such as the temperature, pH value may vary according to different needs of the host cells. Cells are harvested when growing to the appropriate density and disrupted by using any known methods, such as freeze-thaw cycling, sonication, cell lysing agents and mechanical disruption. The CKLF1 polypeptide and variants thereof can be isolated and purified from host cell lysate or extracellular media by methods such as ammonium sulfate or ethanol precipitation, acid extraction, ultra-filtration, anion or cation exchange chromatography, phosphocellulose chromatography, hydrophobic interaction chromatography, hydroxylapatite chromatography, affinity chromatography and highpressure liquid chromatography.

The fourth aspect of the present invention relates to uses of the CKLF1 polypeptide and polynucleotide in the diagnosis and treatment of some diseases. First, the CKLF1 polypeptide has a broad-spectrum chemotaxis to many cells (see Example 6), which implies it can be used to treat some infectious diseases. For example, the chemotactic activity of the CKLF1 polypeptide to inflammatory cells may be used in the preparation of anti-inflammatory drugs containing the CKLF1 polypeptide or its functional fragments to cure autoimmune diseases. Second, the CKLF1 polypeptide exhibits a stimulating activity to the proliferation and colony formation of bone marrow cells, as shown in Example 7 and 8. Thereby, it can be used to stimulate the hematopoietic function of the body and to treat hematopoietic system diseases, including primary and secondary hematopoietic disorders. Third, the CKLF1 polypeptide has the capability of promoting cell proliferation. As showed in Example 10, the CKLF1 polypeptide can stimulate proliferation of skeletal cells and hair follicles *in vivo,* which implies it can be used to treat myophagism or other degeneration diseases. It has been found that in the process of proliferation, skeletal cells are more prone to accept exogenous DNA. Therefore, to promote the absorption of DNA vaccines or drugs, CKLF 1 can be concurrently administrated or injected with DNA vaccines or drugs to improve the immunocompetency of the body and to improve the curative effect. Furthermore, the CKLF1 polypeptide of the present invention can promote proliferation of hair follicular cells and can be used as follicular proliferation agent to cure baldness. Fourth, the expression of the CKLF1 polynucleotide has been found in many tissues and cells, especially in some tumor cells (see Example 9), which implies that the CKLF1 polynucleotide can be used as a diagnostic reagent to detect abnormal CKLF1 gene in some cells.

The detection of a specific DNA sequence may be achieved by methods such as Restriction Fragment Length Polymorphisms (RFLP) and fluorescence *in situ* hybridization (FISH).

The present invention also relates to diagnostic assays for detecting altered levels of the polypeptide of the present invention in various tissues since an over-expression of the protein compared to normal control tissue samples can detect the presence of certain disease conditions such as some primary tumors. Assays used to detect levels of the polypeptide of the present invention in a sample derived from a host include radioimmunoassay, competitive-binding assays, Western blot analysis or an ELISA assay.

The fifth aspect of the present invention relates to mixing the CKLF1 polypeptide or its functional fragment, with pharmaceutically acceptable carrier(s) or excipient(s) to make medical compositions. Such medical composition may be made into various formulations such as solutions, liposome coated formulations, micro-micro-capsules and modified-release formulations, to meet the requirements of administration for achieving advantageous curative effects. Examples of carrier and excipient include saline, isotonic dextrose solution, buffered saline, glycerol, ethanol and combinations othereof. If needed, additives or accessory ingredients may be also added into such composition, for example, reagents showing co-operative effect to CKLF1; protein protective reagents such as human albumin kali and some low-molecular peptides; amino acids such as glycin and lysine; metallic positive ions such as Zn²⁺, Mn²⁺, Mg²⁺ and Ca²; stabilizers such as polyethylene glycol, methylol cellulose, polyglycine and glutathione; proteinase inhibitors; free radical scavengers; skin penetrating reagents (e.g. dimethyl sulfoxide and laurocapram) if locally used at mucosa membrane or skin.

The sixth aspect of the present invention relates to antibodies specific to the CKLF polypeptides. The CKLF1 polypeptide, its functional fragments, or other derivatives, or analogs thereof, can be used as immunogens to produce antibodies thereto. These antibodies can be, for example, polyclonal or monoclonal antibodies. The present invention also includes chimeric, single-chain, and humanized antibodies, as well as antibody-binding fragments (Fab), or that of a Fab library. Various procedures known in the art may be used to produce such antibodies and fragments, for example, the hybridoma technique may be used to produce monoclonal antibodies. Also, transgenic mice may be used to express the humanized antibodies to immunogenic polypeptide products of this invention. Antibodies can be used to isolate the polypeptide from tissue expressing the CKLF1 polypeptide Even antibodies to a fragment of the CKLF1 polypeptide can be used to isolate the whole native CKLF1 polypeptide.

The seventh aspect of the present invention provides an antagonist of the CKLF1 polypeptide which can block or inhibit the activity of the CKLF1 polypeptide. Such antagonist may be employed to produce a medical composition by mixing with one or more pharmaceutically acceptable carrier(s) or excipient(s). By use as a vaccine and introduced into tumor cells, the antagonist of the invention may play a significant role in the treatment of tumors. It is also possible to use antagonist of the present invention to treat autoimmune diseases such as rheumatoid arthritis, allergies, neoplasia and viral infections.

### PREFERED IMBODIMENTS OF THE INVENTION

The following examples are illustrative and are not intended to limit the scope of the present invention.

### Example 1: Isolation of cDNAs from cDNA libraryderived from U937 cell inhibited by IL-10

### 1.1 Cell Culture

U937 cell line was maintained in RPMI 1640 medium containing 100 U/ml penicillin and 100 U/ml streptomycin plus 10% fetal bovine serum. U937 blast cells were propagated every 3-4 days. Cells were collected when the density was approximately 2 × 10⁷ cells, centrifuged and washed three times with Hank's solution, then resuspended in RPMI1640 medium containing 10% FCS and 10 ng/ml PHA. Half of the cells (1 × 10⁷) were used as a tester of SSH; while the other half (1 × 10⁷) were inhibited with 100 ng/ml IL-10 and used as a driver. mRNAs extracted from the tester and the driver U937 cells were used for further DNA synthesis.

### 1.3 Synthesis of the Double-strands cDNA

By using the QickPrep® micro mRNA Purification Kit (Pharmacia), the mRNAs of the tester and the driver were extracted according to the manufacturer's protocol.

By using the reagents and enzymes of the PCR-Select™ cDNA Subtraction Kit (CLONTECH), the single-stranded and double-stranded cDNA were synthesized according to the manufacturer's protocol, in this reaction, the extracted mRNAs of the tester and the driver were used as templates, respectively.

### 1.4 Suppression Subtractive Hybridization (SSH)

A schematic representation of the SSH method is shown in Figurel. The tester cDNA (2µg) and the driver cDNA (2µg) were digested with *Rsa*I. The tester cDNA was then divided into two populations, which were ligated to adapter 1 and adapter 2 in separate reactions by the effect of ligase. The adapter 1- ligated tester and the adapter 2-ligated tester were hybridized with the driver, respectively. After the first hybridization, the resulting samples were mixed and a fresh portion of heat-denatured driver was added for the second hybridization.

The annealed product of the final hybridization was diluted by 1,000-fold and used as the template of the primary PCR. With the primers of the Kit, the primary PCR reaction was performed for 27 cycles.

The amplified product of the primary PCR was diluted by 10-fold and used as the template of the secondary PCR, the nested primers P1 and P2 was added, and the secondary PCR was performed for 15 cycles. Products of the secondary PCR include fragments with different lengths, fragments within the range of 200-1600 bp were recovered and inserted into the pGEM-T easy plasmid (Promega). The recombinant vectors were transformed into XL-1 Blue host cells and white colonies were screened.

### 1.6 DNA Sequencing

Purified plasmids were obtained by using tip-20 Minipreparation Kit (Qiagen), DNA sequencing was carried out on the ALFexpess II DNA sequencer (Pharmacia) at Dalian Baoshengwu Bioengineering Ltd.

### Results

The encoding sequence was obtained by splicing the overlapped express sequence tags W38899, N95062, AA429945, AA987264, AA927461, W19056, N89912, AA516431, AA479657, AA455042, AA989129, W52820 of the EST Assembly Machine (http:// www.tigen.it). As shown in SEQ ID NO: 1, the obtained full-length cDNA of CKLF1 has 534 base pairs, including a poly(A) tail and a polyadenylation signal ATTAAA. The open reading frame is from nucleotide 152 to nucleotide 449, which encodes a polypeptide comprising 99 amino acid residues. The nucleic acid sequence homology was analyzed by GenBank database (http://www.ncbi.nih.gov). The GenBank accession number of the CKLF1 cDNA is AF096895.

Software such as PcGene, Prosite and Signal P server (http://www.cbs.dtu.dk/services/signalP) were used to analysis the amino acid sequence of the CKLF1 polypeptide which has been set forth in SEQ ID NO: 2. The results show that two successive Cys, a character of the C-C chemokine subfamily, is contained in the CKLF1 polypeptide. The first amino acid residue of the deduced mature polypeptide is glycine. No typical signal cleavage site, transmembrane domain, DNA binding site and putative N-glycosylation site have been found in the deduced CKLF1 polypeptide. The first 17 amino acid residues at the N-terminal are hydrophobic and may be the potential leading sequence. The homology analysis showed that the CKLF1 polypeptide shares no obvious homology with known proteins, but shares 46% homology with permease of *Caenorhabditis elegan* from residue 35 through residue 79.

### Example 2: Cloning of CKLF 2, 3 and 4

The following oligonucleotides were designed according to the CKLF1 cDNA.

P1, 5'-ATG GAT AAC GTG CAG CCG AAA AT -3'

P2, 5'-CCG CTC GAG TTA CAA AAC TTC TTT TTT TTC -3'

In the semi-quantitative RT-PCR, cDNAs from the PHA-stimulated U937 library and from the PHA-stimulated, IL-10 inhibited U937 library were used as a template, respectively, oligonucleotides P1 and P2 were used as primers. PCR was performed with the following parameters: 94°C for 2 minutes; 30 cycles at (94°C for 15 seconds, 58°C for 15 seconds, 72°C for 30 seconds); and a final extension at 72 °C for 7 minutes.

### Results

PCR products with different lengths were amplified from two cDNA libraries and cloned into pGEM-T Easy. DNA sequencing were performed as described above. The identified CKLF2, 3 and 4 polynucleotides have been set forth in SEQ ID NO: 3, SEQ ID NO: 5 and SEQ ID NO: 7, respectively. The GenBank accession numbers of which are AF135380, AF135381 and AF145216, respectively.

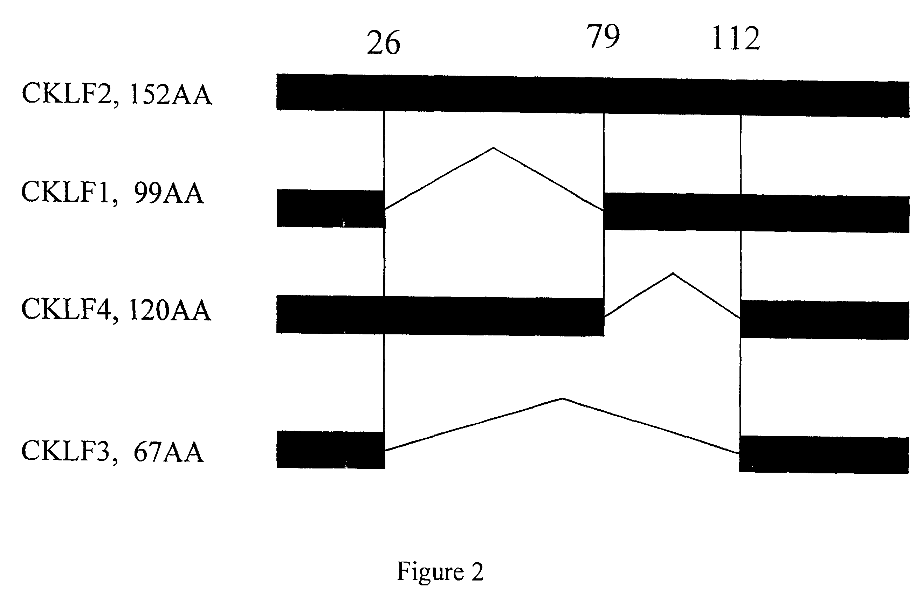

As demonstrated in Figure 2, no frameshift mutation has been observed in the encoding sequence of the CKLF2, CKLF 3 and CKLF 4 polynucleotides compared with that of the CKLF1 (SEQ ID NO: 1). The polypeptides encoded by the CKLF2, 3 and 4 polynucleotides contain 152, 67 and 120 amino acid residues, respectively, as correspondingly set forth in SEQ ID NO: 4, SEQ ID NO: 6 and SEQ ID NO: 8. The CKLF3 polypeptide maintains the conserved amino acid sequence at both the amino-terminal and the carboxyl-terminal, while the CKLF2 and CKLF 4 polypeptides are coded by different exons. According to the result of PcGene analysis, transmembrane domains may exist in the CKLF2 and CKLF 4 polypeptides. It has been assumed that the CKLF2, 3 and 4 polypeptides result from different splicing of the same mRNA.

### Example 3: Detection of the CKLF1 mRNA in U937 cells by Northern blot

The total RNAs of the tester and the driver U937 cells were extracted by using GIBCO-BRL TRI_{ZOL}™ (GIBCO-BRL). 1×10⁷ of the tester and the driver U937 cells were collected by centrifugation, respectively. 1 ml of TRI_{ZOL}™ reagent was added to lyse the U937 cells and 0.2 ml of chloroform was then added. Cells were centrifuged and supematants were collected. The total RNA contained in the supernatants was isopropanol-precipitated, ethanol-washed, and resuspended in RNase-free deionized H₂O and measured with a spectrophotometer (Beckman, 640 nm).

The RNA sample and the 5 × RNA loading buffer were mixed together at the ratio of 4:1 (volume vs. volume). The mixture was incubated at 65°C for 3-5 minutes and then placed on ice immediately. In this experiment, the total RNAs of the tester and the driver (20µg per well) were isolated on a 1.2 % agarose formaldehyde gel under the voltage of 5-7 V/cm, and then transferred onto a GeneScreen-plus nylon membrane by capillary transfer.

By using Random Primer Fluorescein Labeling Kit with anti-fluorescein HRP (DUPONT NEN®, Boston, MA), The full length CKLF1 cDNA obtained by EST splicing was used as the probe of Northern blot. The procedures for probe labeling and sequence hybridization were performed according to the manufacturer's protocol.

### Results

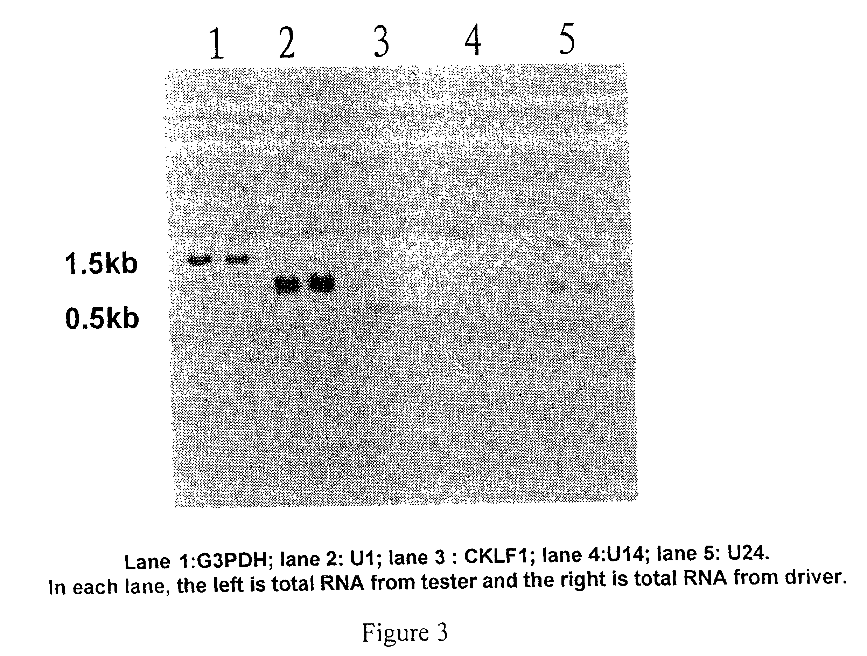

As shown in Figure 3, the length of CKLF1 cDNA is about 0.5 Kb. The expression level of CKLF1 cDNA was high in PHA-stimulated U937 cells, but IL-10 inhibited the expression of CKLF 1.

### Example 4: Expression of the CKLF1 polypeptide in prokaryotic cells

### 4.1 Construction of a Prokaryotic Expression Vector

The oligonucleotides P1' and P2' were designed according to the CKLF1 cDNA and are shown as below. P2' contains the *Xhol*I endonuclease cutting site.

P1', 5'- CTG ATA CCA GAA ACC ACA ACA TT -3'
P2', 5'- GGA AGA ATA CAG AAA TAT GTT TAA TAC -3'

By using the recombinant plasmid pGEM-T easy-CKLF1 as a template, and oligonucleotides P1' and P2' as primers, amplification of the CKLF1 fragment was performed by PCR. The resulting product was blunted by Klenow enzyme and digested with *Xhol*I. The digested CKLF1 fragment was inserted into pMTY4 which had been digested with *Stu*I and *Xhol*I, thus the recombinant plasmid pMTY4-CKLF1 was constructed.

### 4.2 Expression and Purification of the CKLF1 polypeptide

At the temperature of 42°C, the *Escherichia coli* strain pop2136 containing the pMTY4-CKLF1 plasmid was induced to express the MS2-CKLF1 fusion protein in the form of inclusion body. The MS2 and CKLF1 polypeptides were linked by a peptide containing a thrombin cleavage site. Bacteria cells were collected and the inclusion body was then disrupted by sonication. The inclusion body was denatured in denaturation buffer ( 20 mM Glycin-NaOH, 8 M urea, pH 10.0) at 37°C for 30 minutes, and renatured by diluting the concentration of urea to 1 M, the resulting polypeptide was digested overnight at 25°C with thrombin, and purified by ion exchange chromatography with Q Sepharose Fast Flow. The cleaved CKLF1 protein was loaded onto the purification column in 20 mM of Glycin-NaOH (pH 10.0). The column was then washed with 50 mM of Tris-HCl (pH 8.9) and the target protein was eluted by 0.04 M NaCl solution.

### Results

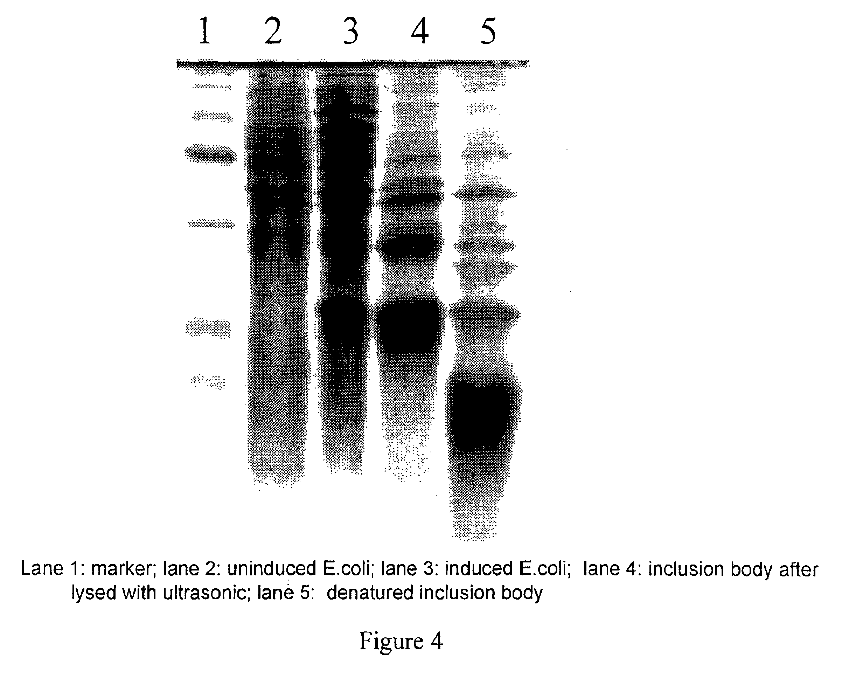

As shown in Figure 4, the expression level of the MS2-CKLF1 fusion protein is high in *E. coli* and the fusion protein takes in about 30 percent of the total proteins expressed by the bacteria. Further, the existence of dimer protein (protein consisting of two polypeptide chains) was observed. The approximate proportion of dimer proteins vs. monomer proteins is 1:4.

### Example 5: Subcellular localization of CKLF1, 2 and 4 in eukaryotic cells

Similar procedures as described in Example 4 were carried out to construct recombinant plasmids containing the CKLF1, 2 and 4 cDNA. The CKLF1, 2 and 4 fragments were amplified by using P1' and P2' as PCR primers. The PCR products were blunted by klenow enzyme and digested with *Xho*I.

The expression vector pEGFP-C3 (CLOTECH) was *EcoR*I-digested, klenow enzyme-blunted and then digested with *Bam*H*Sal*I.

The recombinant plasmid pEGFPC3-CKLF 1 can express a fusion protein consisting of the enforced green fluorescence protein (EGFP) and the CKLF1 polypeptide in eukaryotic cells, the open reading frame of the CKLF1 polypeptide is in immediate succession to that of the EGFP.

Recombinant plasmids pEGFPC3-CKLF1, 2 and 4 were purified and further transfected into Hela cells by using Superfect Transfection Reagent (Qiagen). After incubated for 72 hours, the cells were washed three times with PBS and then fixed in 4% paraformaldehyde for 30 minutes. Cells were washed with PBS again after incubated at the room temperature for 30 minutes. Distribution of the CKLF1, polypeptides were observed by detecting the fluorescence intensity of the fusion protein with a fluorescence microscope.

### Results

In cells expressing the CKLF1-EGFP fusion protein, weak fluorescence was dispersedly detected within the cells. With this result and the following activity detection in supernatants of pcDI-CKLF1-transfected COS-7 cells, it has been concluded that the CKLF1 protein is a secreted one. As for pEGFP-CKLF2 and pEGFP-pEGFP-CKLF 4, fluorescence was mainly detected on cell membranes.

### Example 6: Chemotaxis of CKLF 1

### 6.1 Cells Lines

K562 is a human chronic myelogenous leukemia cell line; U937 is a human promonocytic cell line; TF-1 is human erythroleukemia cell line; HL-60 is a human acute promyelocytic leukemia cell line. HL-60 cells differentiated into neutrophils after incubation in RPMI 1640 medium containing 1.3% dimethyl sulfoxide (DMSO) for about 4 days.

Separation of neutrophils, lymphocytes and monocytes from periphery blood mononuclear cells (PBMC) is performed as follows. 12 ml of periphery blood from a healthy volunteer is mixed with heparin with a final concentration of 25-50 U/ml and 2 volumes of Hank's solution. The mixture was mixing briefly and added into a half volume of Ficoll-Hypaque (1.077 g/cm³±0.002). After centrifuged at 4°C, 2,000 rpm for 25 minutes, the mononuclear cells between the upper plasma and the lower Ficoll-Hypaque reagents were collected and resuspended in RPMI 1640 medium, then incubated at 37°C for 30 minutes. The non-adherent cells are lymphocytes and the adherent monocytes were obtained by digestion with 5% FCS and *0.5%* EDTA. The neutrophils above the erythrocyte layer were purified by lyses with ammonium chloride (pH 7.2) to eliminate the contaminating erythrocytes.

### 6.2 Chemotaxis Assay

The coding sequence of the CKLF1 polynucleotide was released from the pGEM-T easy-CKLF1 plasmid digested with *EcoR*I and subcloned into the *EcoR*I-digested pcDI. A recombinant plasmid pcDI-CKLF1 was thus constructed.

Plasmid pcDI was used as a control, pcDI and pcDI-CKLF1 were transfected into COS-7 cells, respectively. Supernatants were collected and diluted for chemotaxis analysis. The purified cells were adjusted to 1 × 10⁶/ml and loaded into the upper wells of the Boyden chamber. Neutrophils were incubated for 1 hour and other cells were incubated for 3 hours. Then the membrane was taken from the chamber and the nonspecific cells were scraped. Migrated cells were methanol-fixed and Giemsa-stained. Migrated cells in five randomly selected visual fields were counted under a microscope ( magnification: 40×). Chemotaxis of CKLF1 was measured by the mean value of chemotactic index (CI), the ratio of migrated cells in sample group to which supernatants of pcDI-CKLF1-transfected COS-7 cells were added vs. migrated cells in control group to which supernatants of pcDI-transfected COS-7 cells were added.

### Results

As illustrated in Figures 5A-5I, the supernatants of pcDI-CKLF1-transfected COS-7 cells exhibit obvious chemotactic effect on human neutrophils, monocytes, U937 cells, K562 cells, DMSO-stimulated HL-60 cells, mouse peritoneal macrophages and lymphocytes, but exhibit little chemotactic activity on TF-1 cells, NFS-60 cells and non-stimulated HL-60 cells. The above reveals that the CKLF1 protein is secretive and has a broad-spectrum chemotaxis. The chemotaxis of CKLF1 can be completely blocked by a strong reductant DTT. In addition, chemotaxis of CKLF1 exhibits the tendency of dose-dependent.

### Example 7: The enhancing effect of CKLF1, 2 and 3on the proliferation of mouse marrow cells

### 7.1 Construction of Eukaryotic Expression Vectors

The eukaryotic expression vector pcDI was constructed by substituting the *Bgl*II-*Kpn*I fragment of plasmid pCDNA3 (Invitrogen) with the *Bgl*II-*Kpn*I fragment of plasmid pCI (Promega).

The CKLF1 polynucleotide was amplified from PHA-stimulated U937 cell cDNA library with primer P1 and P2 and cloned into pGEM-T. The inserted gene was released after digested by *EcoR*I and was subcloned into the *EcoR*I-digested eukaryotic expression plasmid pcDI. The sense-inserted bacteria clones were screened and the recombinant plasmid was named pcDI-CKLF1, in which the CKLF1 encoding sequence was inserted in right direction. The construction of recombinant plasmids pcDI-CKLF2 and 3 were similar to that of pcDI-CKLF1 (Figure 6).

The purified plasmids were transfected into COS-7 cells by using Superfect Transfection Reagent (Qiagen) and supernatants were collected for biological analysis.

### 7.2 Cell Proliferation Assay Using MTT Method

Bone marrow cells were obtained from BALB/c mice. After lysed with NH₄Cl-Tris (pH 7.2) to remove contaminating erythrocytes, the density of bone marrow cells were adjusted to 1.5×10⁶/ml. 90 µl of cell suspension was plated in each well of a 96-well microplate. 10µl of supernatants of 72-hour-cultured non-transfected COS-7 cells was used as a negative control, while 10µl of solution with 100 ng/ml of IL-3 and 100 ng/ml of SCF was used as a positive control. 10µl of supernatants of COS-7 cells transfected by pcDI, pcDI-CKLF1, 2 and 3 respectively were added to each sample well. Both the sample group and the control group contained 6 wells. Cells were incubated at 37°C, 5% CO₂ for 90 hours. 10 µl of MTT with a concentration of 10 mg/ml was then added to each well. After cells were incubated continuatively for six hours, lysis buffer (50% DMSO, 20% SDS, pH 4.4) was added. By using ELISA-reader, spectrum absorption of cells in different wells was measured at 570 nm.

### Results

As shown in Figure 7, supematants of pcDI-CKLF1 and pcDI-CKLF2 transfected cells exhibit obvious promoting effects on the proliferation of mouse bone marrow cells, while the effect of supernatants of pcDI-CKLF3-transfected cells is weaker. After cells were incubated for 72 hours, no obvious difference was observed in cells within different sample wells, some cells dying naturally. After continuing incubated for 80 hours, some round, medium-sized cells were observed in wells containing supernatants of pcDI-CKLF1 or pcDI-CKLF2-transfected cells, but such cells were scarcely observed in wells containing supernatants of pcDI-CKLF3-transfected cells. Cell shape and quantity in wells containing supernatants of pcDI-CKLF3-transfected cells was similar to that in wells containing supematants of pcDI -transfected cells, as shown in Figures 8A and 8B.

### Example 8: The enhancing effect of CKLF1 on the growth of human low-density bone marrow cells

### 8.1 Stimulating Effect on Cell Proliferation

Low-density bone marrow cells were separated from bone marrow of a healthy volunteer by density cut separation on Ficoll-Hypaque. The separated and purified marrow cells were adjusted to 2 × 10⁶/ml, 90 µl of which were plated into each well in a 96-well microplate. 10 µl of supernatants from pcDI-CKLF1-transfected COS-7 cells were added to each sample well to analyze the effect of CKLF1. 10 µl of supernatants from 72-hour-cultured non-transfected COS-7 cells were used as a negative control, while 10µl of rhGM-CSF with a final concentration of 10 ng/ml were used as a positive control. Cell proliferation was detected by MTT as described above.

### Results

As shown in Figure 9, the 10-fold diluted supernatants of pcDI-CKLF1- transfected COS-7 cells can promote the proliferation of human bone marrow cells. The promoting effect of which is similar to that of rhGM-CSF with a final concentration of 100 ng/ml.

### 8.2 Stimulatory Effect of the CKLF1 Polypeptide on Colony Formation

Low-density bone marrow cells from the health volunteer were adjusted to 5× 10⁴/ml after separated and purified by Ficoll-Hypaque (1.077 g/cm³). The cell suspensions were seeded in 0.3 % soft agar. Supernatants of pcDI-CKLF1-transfected COS-7 cells with different dilution and supernatants of pcDI- transfected COS-7 cells were added to sample wells. RPMI1640 medium was used as a negative control, while GM-CSF with a final concentration of 100 ng/ml was used as a positive control. After two weeks of culturing in 5% CO₂ at 37°C, colonies in the sample group and control group were counted, the cluster that has more than 50 cells is taken as a colony.

### Results

As described in Table 1, the supernatants containing pcDI-CKLF1 can boost the colony formation of human low-density marrow cells and have an obvious cooperative effect with GM-CSF of 100 ng/ml. Among the pcDI-CKLF1-stimulated marrow cells, giant cells are found, as shown in Figure 10, whose survival period is longer than that of cells stimulated with GM-CSF only.

**Table 1.**

| **Stimulating effect of CKLF1 on the granulocyte-monocyte colony formation of human low density bone marrow cells** | | | | | |
|---|---|---|---|---|---|
| Concentration | GM-CSF | pcDI | CKLF1 | GM-CSF +pcDI | GM-CSF+CKLFI |
| 10⁻¹ | 22±3 | 11±2 | 28±3 | 21±2 | 36±3 |
| 15⁻¹ | | 11±1 | 16±2 | 23±1 | 28±2 |
| 30⁻¹ | | 10±3 | 10±2 | 20±2 | 22±2 |

### Example 9: Expression of CKLF1 and its variants in normal and tumor tissues

The expression level of CKLF1 and its variants in fetal, adult and tumor tissues was analyzed by using Multiple Tissue cDNA Panel (Clontech). cDNA of the one-stranded cDNA library provided by the manufacturer were used as a template, P1 and P2 were used as primers. The PCR reaction was performed under the same condition as described above.

### Results

As illustrated in Figures 11A and 11B, the expression level of CKLF1 and its variants was low in tissues of healthy adult and could hardly be detected in prostate and pancreas tissue. However, highly expressed CKLF1 and CKLF2 were detected in fetal tissue and many tumor tissues, such as breast cancer, colon cancer, lung cancer, ovarian cancer, pancreatic cancer and prostate cancer, while the expression level of CKLF3 and CKLF4 in those tissues is much lower (Figure11C-11D). These results indicate that CKLF1 may have a close relationship with embryonic development and tumorgenesis.

### Example 10: Biological activity of CKLF1 in vivo

BALB/c mice of 4-6 weeks were purchased from the Genetic Institution of China Academy of Medical Science. Eukaryotic expression plasmids pcDI and pcDI-CKLF1 were purified by tip-10000 Giga-preparation Kit (Qiagen). In the pcDI-CKLF1 group, 100 µl of pyrogen-free saline containing 100µg of pcDI-CKLF1 was injected to each mouse at anterior tibial muscle tissue and subcutaneous tissue. In the control group, each mouse was injected with the same saline containing 100µg of pcDI. Bupivacaine or electric pulse (40ms, 80v) was used to enhance the absorbance of DNA. On the 10th and the 30th day after injection, mice were killed and the injection sites were excised. Samples of muscle biopsies were analyzed by staining with Hematoxylin-Eoxin (HE), Fuelgen and Acid Phosphatase (AcP), respectively, and then observed under a microscope. The biological effect of CKLF1 on the injected tissues was observed by the method of histological, immunohischemical and enzymological analysis.

### Results

The function of the pcDI-CKLF1 polypeptide is: (1) attracting inflammatory cells to the pcDI-CKLF1-injected sites; (2) boosting proliferation and differentiation of skeletal muscle cells; (3) promoting proliferation and differentiation of keratinocytes of follicles.

The results of AcP (Figure12A-12B) and HE staining (Figure13A-13B) demonstrated that, compared with normal skeletal muscle cells, cells in the control group to which 0.9% saline containing pcDI was injected have no obvious shape or morphology change. But in the pcDI-CKLF1 sample group, more nuclei appeared in the middle of the muscle fiber tissue, ranked like a string of beads, and were accompanied by the phenomenon of monocyte-macrophage infiltration. Such phenomenon was more remarkable at 10, 20 and 30 days after injection, especially at 10 days after injection.

By using Leica Q500MC analyzer, the result of Feulgen staining was analyzed. The mean optical density (MOD) of cells in the pcDI-CKLF 1 sample group was much higher than that in the pcDI control group (Table 2), which indicates that there are more nuclei accumulated in muscle tissue. The increase of nuclei was more evidently observed in muscle cells injected with pcDI-CKLF1 plus needling (t test p<0.01).

**Table 2.**

| **Result of Feulgen staining of the injected skeletal muscular tissue (x ± s, unit MOD)** | | | | |
|---|---|---|---|---|
| plasmid | pcDI-CKLF1 (sample group) | | pcDI (control group) | |
| group | A | B | A | B |
| quantity of nucleus | 514.33±32.18 | 666.7±42.51 | 67.18±11.21 | 63.43±14.34 |
| MOD of nucleus | 0.134±0.017 | 0.118±0.023 | 0.098±0.008 | 0.094±0.001 |
| Note: A is bupivacaine injection + muscle injection (IM), B is IM + needling | | | | |

## Claims

1. A chemokine-like factor 1 polypeptide as set forth in SEQ ID NO: 2 and functionally identical fragments, variants or derivatives thereof.

2. The polypeptide of claim 1 having the amino acid sequence encoded by the plasmid contained in CGMCC Deposit NO.0392.

3. A variant of the chemokine-like factor 1 polypeptide having the amino acid sequence selected from the group consisting of SEQ ID NO: 4, SEQ ID NO: 6 and SEQ ID NO: 8.

4. An isolated polynucleotide encoding the chemokine-like factor 1 polypeptide, or an allele variant of said polynucleotide, or a polynucleotide capable of hybridizing to said polynucleotide under stringent conditions.

5. The polynucleotide of claim 4 having the sequence as set forth in SEQ ID NO: 1.

6. The polynucleotide of claim 4 or 5 wherein the polynucleotide is genomic DNA.

7. The polynucleotide of claim 4 or 5 wherein the polynucleotide is cDNA.

8. The polynucleotide of claim 4 or 5 wherein the polynucleotide is RNA.

9. A vector containing the polynucleotide as claimed in any one of claims 4 - 8.

10. A host cell containing the vector of claim 9 wherein said host cell may be transformed, transfected or transducted with the vector of claim 9.

11. A method for producing the polypeptide of claim 1 comprising introducing a vector containing the chemokine-like factor 1 polynucleotide into a host cell by recombinant techniques, culturing the host cell under suitable conditions, and obtaining said polypeptide from cell lysate or extracellular media.

12. A medical composition comprising a therapeutically effective amount of the chemokine-like factor 1 polynucleotide or functional fragment thereof, and one or more pharmaceutically acceptable carriers and/or excipients.

13. A monoclonal or polyclonal antibody wherein said antibody may specifically combine with the chemokine-like factor 1 polypeptide.

14. An antagonist which inhibits activation of the chemokine-like factor 1 polypeptide.

15. A diagnostic method *in vitro* comprising detecting the presence of the polypeptide of claim 1 or the polynucleotide of claim 2 in a sample derived from a host.

16. A method of using the polypeptide of claim 1 or the polynucleotide of claim 2 in the preparation of immunological adjuvants capable of improving the curative effect of a DNA vaccine or a DNA drug.

17. A method of using the polypeptide of claim 1 or the polynucleotide of claim 2 in the preparation of medical compositions capable of treating inflammations, degeneration diseases, primary tumors and hematopoeitic disorders.

18. The method of claim 14, wherein said inflammations comprise rheumatoid arthritis and other autoimmune diseases, allergic and hyperplastic diseases.

19. The method of claim 14, wherein said degeneration diseases comprise muscular dystrophy and hair loss.

20. The method of claim 14, wherein said tumors are selected from the group consisting of the breast cancer, colon cancer, lung cancer, ovary cancer, pancreas cancer and prostate cancer.
